(19) Europäisches Patentamt

European Patent Office

Office européen des brevets

(11) **EP 0 670 491 B1**

(12) **EUROPEAN PATENT SPECIFICATION**

(45) Date of publication and mention
of the grant of the patent:
**21.11.2001 Bulletin 2001/47**

(51) Int Cl.⁷: **G01N 33/18**, C12Q 1/58

(21) Application number: **95500023.7**

(22) Date of filing: **01.03.1995**

(54) **Method and device of measuring the concentration of total ammonium in a liquid medium**

Verfahren und Vorrichtung zur Konzentrationsmessung des totalen Ammoniums in einem flüssigen Medium

Procédé et dispositif pour mesurer la concentration d'ammonium total dans un milieu liquide

(84) Designated Contracting States:
**AT BE CH DE ES FR GB IT LI NL SE**

(30) Priority: **02.03.1994 ES 9400417**

(43) Date of publication of application:
**06.09.1995 Bulletin 1995/36**

(73) Proprietor: **FUNDACIO AGBAR, CENTRE D'ESTUDIS I INVESTIGACIO DEL MEDI AMBIENT**
**08013 Barcelona (ES)**

(72) Inventors:
• **Alegret Sanroma, Salvador**
  **E-08024 Barcelona (ES)**
• **Alonso Chamorro, Julian**
  **E-08026 Barcelona (ES)**
• **Bartoli Molins, Jordi**
  **E-08193 Bellaterra (ES)**
• **Paulis Fernandez, Josep Ma**
  **E-08018 Barcelona (ES)**
• **Martinez Fabregas, Esteban**
  **E-08025 Barcelona (ES)**

(74) Representative:
**SUGRANES - VERDONCES - FERREGÜELA**
**Calle Provenza, 304**
**08008 Barcelona (ES)**

(56) References cited:
| | |
|---|---|
| EP-A- 0 352 717 | EP-A- 0 359 158 |
| WO-A-89/04967 | DE-A- 2 624 384 |
| DE-A- 3 842 068 | FR-A- 2 255 602 |
| US-A- 3 542 649 | US-A- 4 311 789 |

# EP 0 670 491 B1

**Description**

## Technical field.

This invention refers to a method of carrying out the measuring of the concentration of total ammonium in a liquid medium, in a continuous, automatic and autonomous way, and a device for carrying out this method.

The method and the device which are the object of this invention may be used for measurements carried out in watery mediums of different origin and with very wide concentrations of the pollutant.

## State of the art.

The determination of the total ammonium in a liquid medium is of great interest in diverse fields, among which we could mention water analysis and medicine.

The presence of ammonia in stagnant water, in river beds or water destined for domestic consumption may cause limitations in use and force measures which must be known a priori.

This presence of ammonia in water, basically has its origin in domestic or farming faecal waste, therefore the importance of its detection is obvious due to the consequences it can bring about in consumption.

The treatment of this pollutant by chlorination may produce chloramines, which interfere with the disinfecting action of the chlorine and are toxic to a certain degree for the human organism.

In river beds, ammonia is specifically toxic for the ichthycolous life, restricting it, and in stagnant waters it can be considered as nutrient nitrogen together with nitrites and nitrates (degradable compounds by oxidation), which can cause eutrophication.

The measurement of the concentration of total ammonium is justified, therefore, because of its origin, which detects the presence of faecal pollution, as well as the use of the water, which could need a specific treatment or limitations, which has caused the approval of the regulations in force in some countries in the European Union, which fixes the limits of domestic water purifier waste.

In the field of medicine, the continuous measurement of urea is of vital importance for patients with signs of renal failure, as their lives can only be normalised by a kidney transplant which would compensate for the failure of their own. In many cases when this transplant is not possible, it is necessary to carry out a purification of the blood by periodical haemodialysis processes.

The main problem of the aforementioned haemodialysis processes is that, until now, no personalised follow-up is carried out of the quality of the dialysis at each session, so that the needs of each patient are usually defined by supposed parameters and behaviours, and consequently, treated in excess as a safety measure. This means a loss of the quality of life for these renal patients, and this could be diminished by a real time check on some of the parameters such as the grams of urea lost at each moment, blood clearing, KT/V, etc, as this data would allow the processes to be carried out in better conditions and the duration of the analysis for each session would be the best for each patient.

The difficulty in carrying out this follow-up is due to the fact that the urea testers for clinical use are usually conventional robotised spectrophotometric systems. This provides a good sampling capacity for discrete samples, but makes continuous analysis difficult owing to the high cost of apparatus and reagents, the difficulties in managing the liquids for treating the sample, the amount of space they take up, etc.

Because of all of this, several $NH^+_4$ measuring methods have been set up in laboratories as well as on an industrial level by classical stoichiometric methods, in automatic analysis equipment and evaluation equipment.

A group of the automatic analysis equipment developed is based on colorimetric techniques using the detection of the colour of the sample after an adequate colorimetric reaction. This analysis startup needs filtering of the sample which eliminates the particles which could produce refractions of luminous rays during analysis but this need is not always possible in unknown samples. This fact limits the use of this method in current tests.

Another group of automatic analysis equipment is based on techniques using ion selective electrodes, which are sensitive to certain ions, in this case to the ammonium ion and which generate an emf which is proportional to the logarithm of the concentration when in contact with a solution which contains them, and which can be detected and measured by potentiometric methods.

The limits of the use of this equipment concerns the lifetime of the electrode, which is susceptible to contamination when in continuous contact with the sample while the test is being carried out. This is even more the case when real samples sometimes add pollutants which degrade the sensitivity of the electrode relatively quickly. This technique has variations based on the function of the electrode which can be sensitive to pH or to compounds created by adding specific reagents used in each case.

**Description of the invention.**

The method of measuring the concentration of total ammonium or urea by its prior transformation into ammonium in a liquid medium, which is the object of this invention, eliminates the drawbacks mentioned of known-methods and, in essence, is characterised by the fact that in a first cycle of calibration or analysis a mixture of an alkalinizing solution and a standard-composition or a sample of liquid to be analyzed is passed through the face of a semipermeable membrane respectively, while on the other face of the aforementioned semipermeable membrane a solution of an organic buffer compound is made to circulate so that the mixture gives off ammonia in a gas phase, which on passing through the semipermeable membrane is reconverted into ammonium ion, which is passed through an electrode sensitive to the ammonium ion. For example one of the electrodes which are the object of the patent ES-P8803619 and ES-P8803675, which generate an emf which is proportional to the logarythm of concentration of the ammonium ions present, and this potential is compared to that obtained from a reference electrode, after which the difference of potential measured between both electrodes is amplified and converted into an electric signal, which is suitably modified for viewing. The sample is then passed through the circuit and in a second successive, immediate washing cycle, a washing agent replaces the standard composition or the liquid sample, so that the system is prepared for a new cycle of calibration and analysis, followed by the subsequent washing cycle.

According to another feature of the invention, the described device for carrying out the method includes a test circuit which has intake means for the liquid sample to be analyzed; containers means for the washing agent, alkalinizing solution, organic buffer compound solution and calibration standard solutions; impulsion means for the washing agent, alkalinizing solution, liquid sample and organic buffer compound solution; first and second means of selection; a mixture chamber; a semipermeable membrane with an appropriate holder; an electrode which is sensitive to the ammonium ion; a reference electrode; electronic means for treating the electrical signal; and means for representing the treated signal, all located so that in the first cycle of calibration or analysis, the first selection means take a small amount of alkalinizing solution and a small amount of calibration standard solution or liquid sample respectively from the aforementioned containers means, which are then sent by impulsion to the mixture chamber where they are mixed. After this they are passed through the semipermeable membrane, the sensitive electrode and the reference electrode, detecting a difference in the potential which gives an electric signal. This signal is suitably treated by the electronic means and sent to the representation means where the concentration of total ammonium resulting from the analysis is digitally represented. A washing cycle follows immediately, in which a washing agent is passed through the circuit in place of the calibration solution or liquid sample, the device is then ready to perform a new cycle of calibration and analysis, followed by the subsequent washing cycle.

In accordance with another feature of the invention, the aforementioned first and second means of selection are each made up of two-way or more way valves.

According to another feature of the invention, the aforementioned impulsion means are preferably made up of a multichannel peristaltic pump.

In accordance with another feature of the invention, the aforementioned alkalinizing solution is an alkaline metals or alcali-earth hydroxide, with a concentration in the solution greater than 0.1 M.

According to another feature of the invention, the aforementioned organic buffer solution is made up of weak monofunctional bases with a $pK_{acidity}$ between 5 and 8.2 and a concentration in the solution of between 0.01 M and 0.15 M.

In accordance with another feature of the invention, the aforementioned calibration standard solutions are made up of any salt, or combination of salts which contain the ammonium ion, particularly ammonium chloride.

According to another feature of the method of the invention, applicable to measuring urea in watery solutions in the event that the sample that must be analyzed contains ammonia in the form of urea. This sample is passed first continuously through a reactor which contains the urease enzyme, covalently immobilised in a suitable support, in a carrier solution which maintains a pH of between 7 and 8, so that the urea contained in the sample is degraded into ammonium.

In accordance with another feature of the invention, the aforementioned carrier solution is a pH regulator solution adapted to maintain a pH in the order of 7.4.

According to another feature of the invention the aforementioned pH regulator solution is made up of potassium phosphate-potassium hydrogen-phosphate, or by sodium carbonate-bicarbonate.

In accordance with another feature of the invention, the urea to ammonium degradation reaction system incorporates a relatively high flow of sodium hydroxide solution.

According to another feature of the invention, the reactor is preferably made up of a tube of plastic material, adapted to contain the urease enzyme covalently immobilised on its internal walls.

## Brief description of the drawings.

The enclosed drawings illustrate, as a non-limiting example, one way of carrying out the object of this invention.

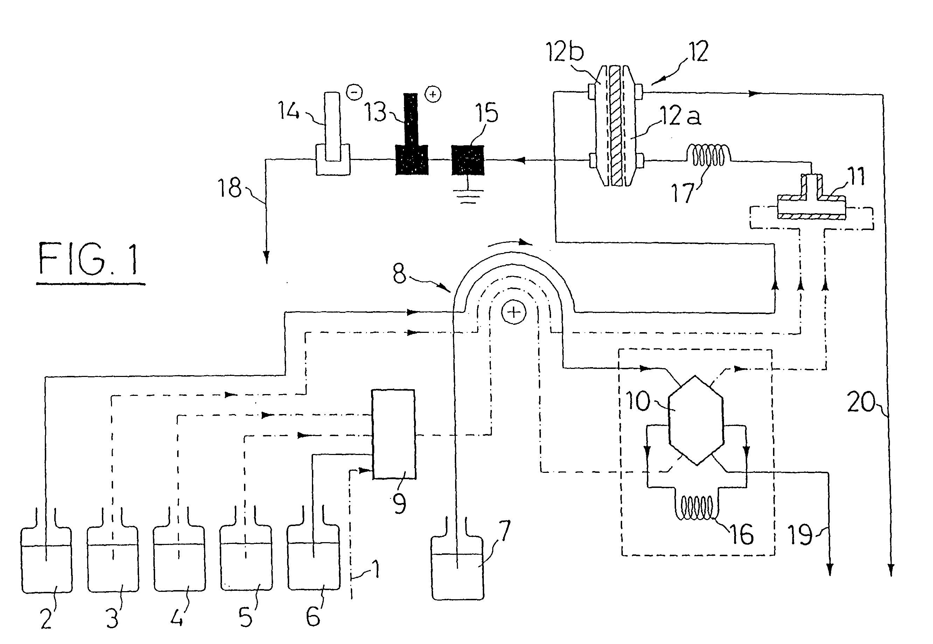

Fig. 1 is a flow chart of the method which is the object of this invention.

Fig. 2 is a diagram of blocks of the device control circuit which is the object of the invention.

Fig. 3 is a flow chart illustrating the method of the invention, with the application to the urea measurement.

Fig. 4 shows a calibration curve in an analysis of the amount of ammonium in a water sample.

Fig. 5 shows the response curve of the electrode in the non-linear area.

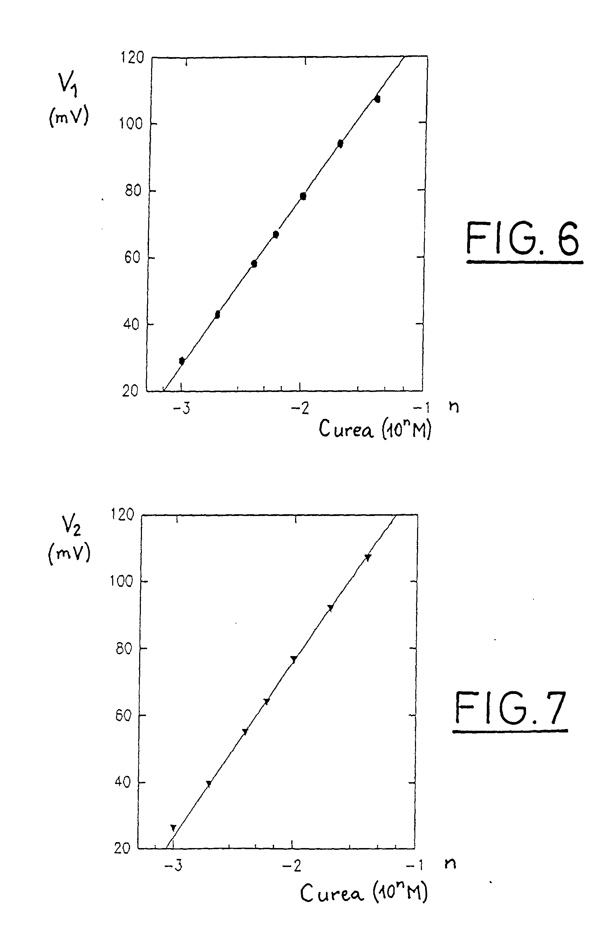

Figs. 6 and 7 both show response curves of the electrode to different concentrations of urea in two cases of measuring urea.

Fig. 8 shows a curve indicating the monitoring of a haemodialysis process.

The drawings show how to carry out the method which is the object of this invention and how the device works. In accordance with the invention, the equipment consists of a liquid sample intake (1), a washing agent tank (2), an alkalinizing solution tank (3), three standard composition tanks (4, 5, 6) and a organic buffer compound solution tank (7).

The first selection means (9), made up of a six-way selector electrovalve, selects the liquid sample or a small amount of one of the three standard solutions and send it to the peristaltic pump (8) which is the means of impulsion.

The impulsion pump (8) is also adapted to impulse the solution of the organic buffer compound solution contained in tank 7, the alkalinizing solution contained in tank 3 and the washing agent contained in tank 2.

The alkalinizing solution and the washing agent reach the mixture chamber (11) directly along the second selection means (10), made up of a six way electrovalve. In this mixture chamber (11) the alkalinizing solution and the washing agent or standard solution or liquid sample to be analyzed are mixed together, the mixture leaving the mixture chamber (11) which reaches one of the faces (12a) of the semipermeable membrane (12) and the solution consisting of the organic buffer compound from tank 7 reaches the other face (12b) of the semipermeable membrane (12).

The mixture from the mixture chamber (11) which has already passed through the semipermeable membrane (12) is now passed through a receptacle (15) connected to an earth point and then, first of all, it reaches an electrode (13) sen-sitive to the ammonium ion and finally a reference electrode (14), then it is emptied down the drain along pipe 18. The product left over from the second elecrovalve (10) is emptied down the drain along pipe 19. As regards the organic buffer compound, after passing through the membrane (12), it is also emptied down the drain along pipe 20.

The equipment also has a first and second loop, 16 and 17 respectively, adapted so as to determine the work volume of the different compositions which are handled by the electrovalve (10) and by the semipermeable membrane (12), and to assure a complete mixing of liquids (17).

Between the sensitive electrode (13) and the reference electrode (14), a difference of potential is detected which gives off an electrical signal which is suitably treated by the electronic means and sent to the representation means so as to provide a digital representation of the concentration of total ammonium in the liquid sample analyzed.

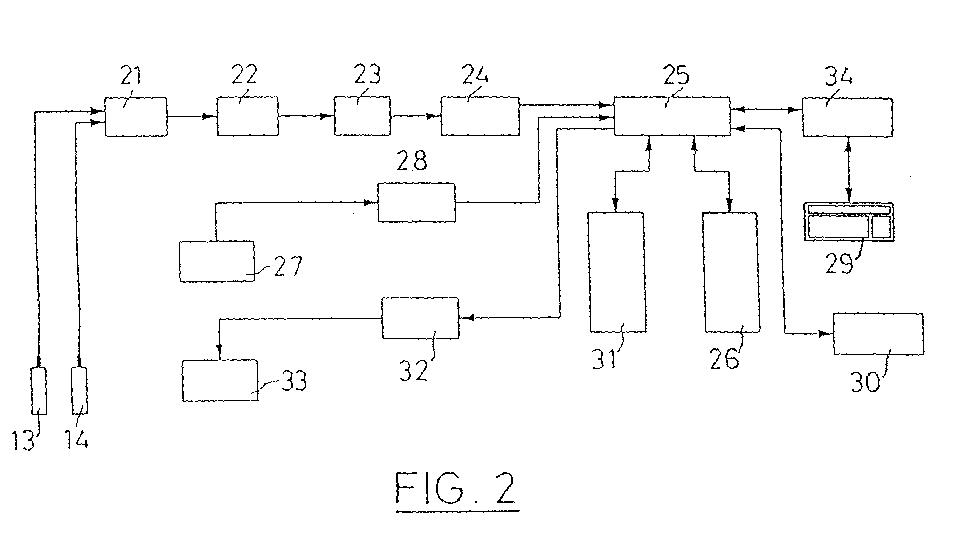

The block diagram in Fig. 2 shows the functional elements which make up the aforementioned electronic and representation means and their relationship. In the diagram both electrodes, the sensitive one (13) and the reference one (14), are connected to a differential amplifier (21), whose output, which is in accordance with the difference in potential which exists between both electrodes 13 and 14, is injected to the input of a voltage amplifier (22) whose output is introduced into a voltage adaptation circuit (23) which processes the resulting output signal for the digital-analogue converter (24) which receives it, being the output of the converter (24) connected to the microprocessor (25) which controls the working of the device by the working program (26). Apart from the data input from the digital-analogue converter (24), which is in accordance with the difference in potential which exists between the sensitive electrode (13) and reference electrode (14), the microprocessor (25) receives: (a) data referring to the level of reagent obtained by a probe (27) which output signal it is adapted by means of a converter (28); (b) data introduced by an operator through a console (29); (c) data from a communications modem (30); and (d) data stored in a reading-writing memory (31).

From the data generated by the electrodes 13 and 14 and by the level probe (27) and the data supplied through the console (29) and the communication modem (30), the microprocessor (25) by means of the working program (26) controls the working of the peristaltic pump (8), by a converter (32) and an operation circuit (33). At the same time, the microprocessor (25) supplies data on the process to a viewing unit (34) and to the communications modem (30), thus providing the operator with permanent information about the measurement process of the sample's ammonium concentration .

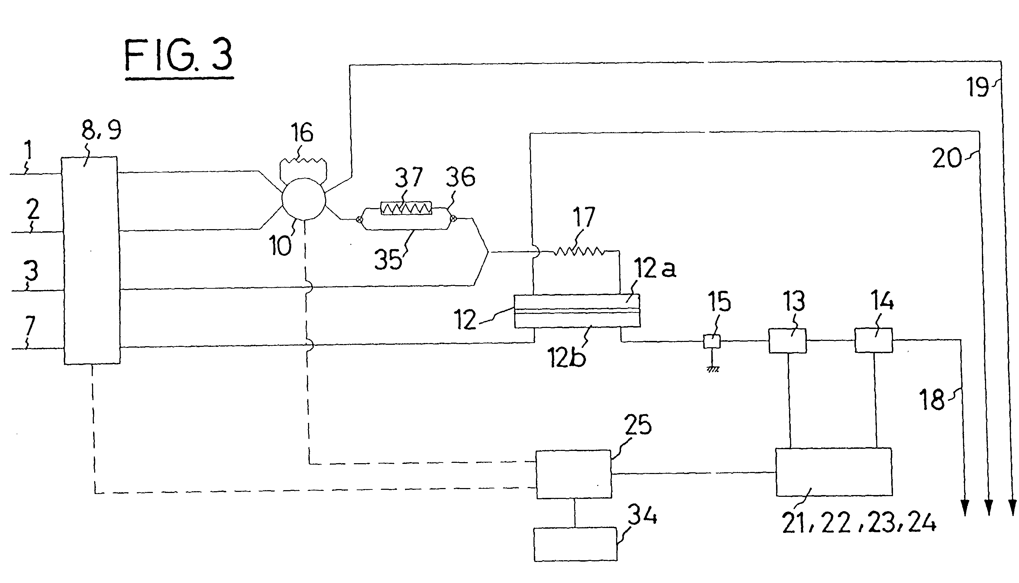

Fig. 3 shows a flow chart of a measurement method, similar to that illustrated in Fig. 2 but adapted so as to perform the measurement of urea. To this end, a parallel, two way circuit is used so that the way (35) connects the electrovalve (10) directly to the second loop (17) and way (36) includes a reactor (37) where the urea is reduced to ammonia.

Next the data of the different reagents is given from electrodes 13 and 14 and from the reactor (37).

Calibration Standard solutions.

Preferably three standard solutions are used, according to the desired range of measurements. Two of the aforementioned standard compositions have concentrations of ammonium between the linear margin of the response of the electrode and the third standard composition in the non linear part of the response curve. The linearity limits of the electrode correspond to concentrations of ammonium between 1E-06M and 1E-02M.

The electrode response in the linear part follows the Nerst equation and the non linear one follows the following equation:

$$E = E_o + g \log (\underline{a} + c)$$

where c is a constant to be determined with the third standard composition and $\underline{a}$ is the concentration of ammonium ions in the sample.

Some possible standard composition concentrations are the following:

$$P_2 > P_1 > P_3$$

$$P_2 > 10 \text{ ppm ammonium}$$

$$P_3 < 0.15 \text{ ppm ammonium}$$

The standard compositions are preferably prepared from ammonium chloride.

Organic buffer compound.

This is a buffer solution prepared from an organic compound, selected especially from among weak multifunctional bases having a $pK_{acidity}$ near to the working pH (between 5 and 8.2) As a suitable compound it could be suggested preferably tris (hydroxymethyl) aminomethane.

The concentration of the organic compound in the solution must be greater than 0.01M, preferably between 0.01 and 0.15M, so as to maintain a suitable buffer capacity.

Alkalinizing solution.

This is prepared from any alkaline metals or alcali-earth hydroxide, in particular from sodium hydroxide. The hydroxide concentration in the solution must be greater than 0.1M.

Washing agent for water analysis.

Distilled water is used.

Washing agents for urea measurements.

A solution of 0.1M of potassium hydrogenophosphate or also a solution of 0.1M of sodium bicarbonate can be used. The pH must be between 6 and 8.6.

All the solutions should be prepared with bidistilled water.

Preparation of the chamber which contains the semipermeable membrane.

This is constructed preferably of two blocks of methacrylate where one of the faces has a 0.2 to 0.4 mm deep groove, along which the solutions will pass. The shape of the groove may be linear or spiral, with a interchange surface of between 1 and 26 cm². The separation of the gas is carried out through a hydrophobic membrane permeable to gas. The membrane is preferably made of ethylene polytetrafluoride or polyvinylidene fluoride. The size of the pore varies according to the application, between 0.22 μm and 3.0 μm. The thickness of the membrane varies between 60 and 250 μm. Commercial membranes of these characteristics are manufactured by the firms MILLIPORE, CLEANOSE, WHATMAN, SARTORIUS, SCHLEICHER & SCHVELL. The Durapore (GV) membranes from Millipore are especially suitable.

<u>Urea reactor.</u>

This is prepared in a tubular shaped nylon support with an internal diameter greater than 1 mm and between 25 and 100 cm long, according to the performance required and to the flow rate employed.

The urease enzyme used is UREASE E.C. 3.5.1.5.

The urease is immobilised by activating the surface of the tube with dimethyl sulphate, then introducing hexamethylendiamine and finally glutaraldehyde before the impregnation of the urease.

Table I shows the flows of each reagent.

TABLE I

| | MEASUREMENT TYPE | |
|---|---|---|
| | Ammonium | Urea |
| Distilled water | 0.8 to 1.2 ml/min | --- |
| Buffer for urea | --- | 0.8 to 1.4 ml/min |
| Sample | 0.8 to 1.4 ml/min | 0.6 to 1.8 ml/min |
| Alkalinizing solution | 0.2 to 0.5 ml/min | 0.4 to 0.8 ml/min |
| $NH_3$, gas receptor solution | 0.5 to 0.8 ml/min | 0.5 to 1.2 ml/min |
| Volume of sample injected | 50 to 1500 $\mu$l (*) | 250 to 1000 $\mu$l(*) |
| Mixing time | --- | --- |
| Sample - alkalinizing solution after injection | 3 to 7 s. | 2 to 6 s. |

(*) Depends on the range the device is working at.

Reference electrode.

Commercial double connection Ag/AgCl or calomel reference electrodes are used, which are usual in potentiometric measurements, as well as identical electrodes to ion-selective ones, passing ammonia receptor solution along it continuously between the latter and the sample to be analyzed.

Calibration process.

As follows it will be explained how to carry out the calibration process according to the method of the invention.

The standard composition $P_1$ which comes from tank (4) is selected by the selection valve (9) for the sample channel and it is pumped until it fills the injection loop; meanwhile, distilled water (2) and the alkalinizing solution (3) is injected along the injector valve (10) of the measurement circuit into the gas diffusion chamber which encloses the semipermeable membrane (12) (sample circuit 12a), at the same time as the organic buffer compound is passed along the measurement circuit (12b).

In these conditions, the voltage between electrode 13, sensitive to ammonium ions and the reference electrode (14) is measured and taken as the reference voltage $V_r$.

Once the time needed to fill the injection loop (16) has elapsed, injector valve 10 is switched so that the measurement circuit injects the standard $P_1$ in place of the distilled water. The voltage is then measured between electrode 13 and electrode 14 $Vp_1$. Choose one of the electrodes described in patents ES-P8803619 and ES-P-8803675 as the electrode sensitive to ammonium ions.

The cycle is repeated with the standard compositions $P_2$ and $P_3$ from 5 and 6, obtaining responses from electrode 13 to these concentrations known as $Vp_2$, $Vp_3$.

With the values $Vp_1$, $Vp_2$, and $Vp_3$ the constants Eo and g are obtained in the linear part of the electrode response equation:

$$E = Eo + g \log \underline{a}$$

While the value $Vp_3$ obtains c in the non linear part of the electrode response equation.

$$E = Eo + g \log (\underline{a} + c)$$

Once the equipment has been calibrated, it is ready for measuring ammonium or urea in test samples.

Measurement of ammonium.

Washing agent: distilled water 2.
Way 35 is opened and way 36 closed.
The same process as that given for the standard composition $P_1$ is followed and an electrode response voltage is obtained $V_m$ which replaced in the last equation gives the concentration $\underline{a}$.

Measurement of urea.

Washing agent: urea 7 buffer solution.
Way 36 is opened and way 35 is closed.
The process is identical to that explained for the measurement of ammonium, and the concentration of ammonium equivalent to urea is obtained in the unknown sample.

Results obtained.

1) In the measurements of ammonium, the results shown in Table II have been obtained. Column A shows the concentration of ammonium in ppm and column B shows the electrode response voltages in mV.

TABLE II

| | A (ppm) | B (mV) |
|---|---|---|
| 1 | 0.018 | 1.6 |
| 2 | 0.036 | 2.6 |
| 3 | 0.09 | 6.7 |
| 4 | 0.18 | 12 |
| 5 | 0.36 | 20 |
| 6 | 0.9 | 38.7 |
| 7 | 1.8 | 54 |
| 8 | 3.6 | 70 |
| 9 | 9 | 94 |
| 10 | 18 | 112 |
| 11 | 36 | 130 |
| 12 | 90 | 152.7 |
| 13 | 180 | 170.7 |

Reproducibility of results: R.S.D. < 2%

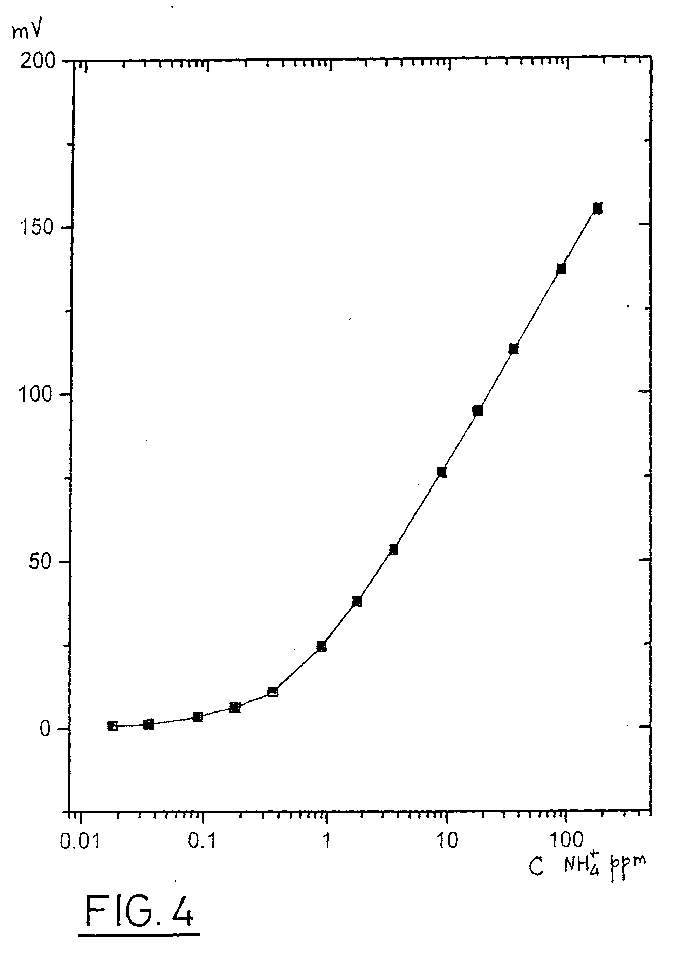

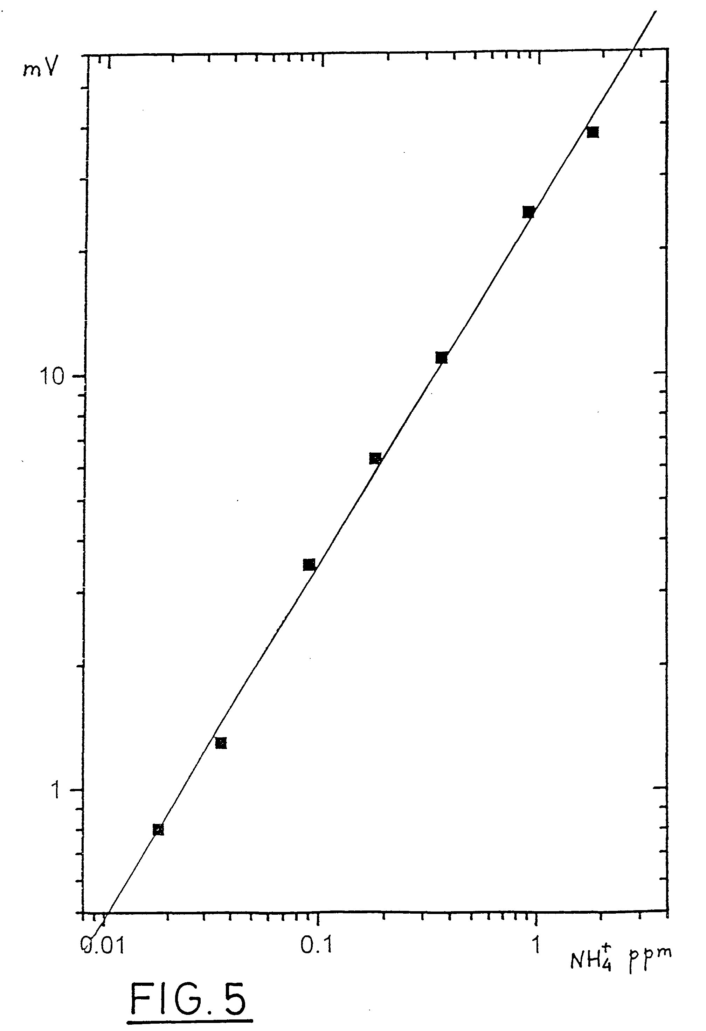

The results are shown in the curves illustrated in Figs. 4 and 5 in the drawings.

2) In the measurements of urea the results shown in Table III are obtained.

TABLE III

RESULTS OF THOSE CALIBRATED

| C.UREA (mol/1) | V1 (mV) | REGRESION1 ANALYSIS |
|---|---|---|
| 0.001 | 29.04 | Intercept 180.005 |
| 0.002 | 42.89 | Slope. 50.84 |
| 0.004 | 58.25 | Corr. Coef. 0.99997 |
| 0.006 | 66.93 | Bottom L. 0.002 |
| 0.01 | 78.21 | Top L. 0.02 |
| 0.02 | 93.83 | |
| 0.04 | 107.34 | |

| C.UREA (mol/1) | V2 (mV) | REGRESION2 ANALYSIS |
|---|---|---|
| 0.001 | 26.15 | Intercept 181.8 |
| 0.002 | 39.39 | Slope 52.82 |
| 0.004 | 55.00 | Corr. Coef. 0.99988 |
| 0.006 | 64.07 | Bottom L. 0.002 |
| 0.01 | 76.61 | Top L. 0.02 |
| 0.02 | 91.96 | |
| 0.04 | 106.84 | |

The graphs shown in Figs, 6 and 7 display the electrode response to different concentrations in two different cases.

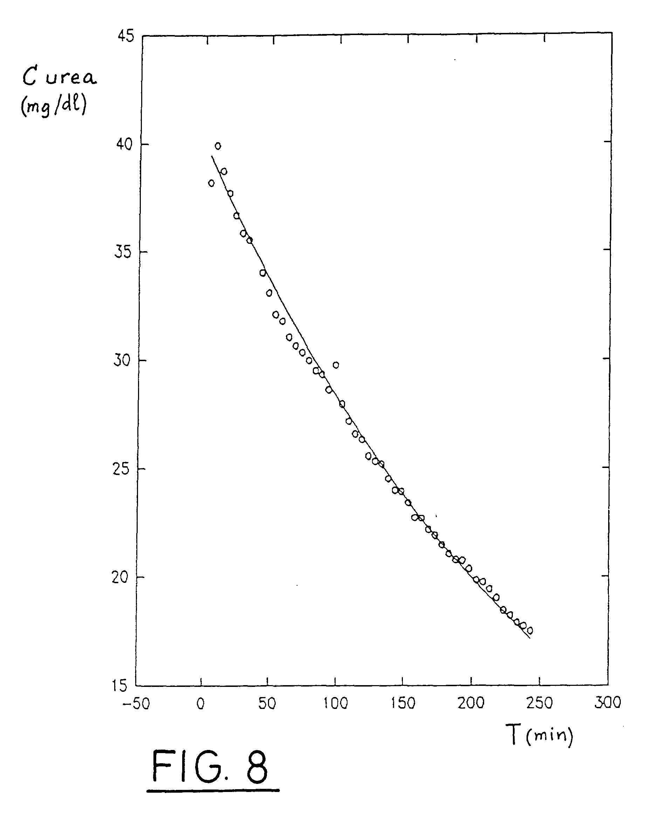

Finally, Fig. 8 illustrates a curve which shows a monitoring of a haemodialysis process in a patient, by the method which is the object of this invention, showing the urea controlled according to time.

## Claims

1. Method of measuring the concentration of total ammonium in a liquid medium, **characterized in that** in a first calibration or analysis cycle a mixture of an alkalinizing solution and a calibration standard solution or a sample of liquid to be analyzed is passed through the face (12a) of a semipermeable membrane (12) respectively, while through the other face (12b) of the aforementioned semipermeable membrane a solution of an organic buffer compound is made to pass, so that the mixture gives off ammonia in a gas phase, which on passing through the semipermeable membrane is reconverted into ammonium, which is passed through an electrode (13) sensitive to the ammonium ion, which generates a potential proportional to the logarythm of concentration of ammonium ions present, and this potential is compared to that obtained form a reference electrode (14), after which the difference of potential measured between both electrodes is amplified and converted into an electrical signal, which is suitably modified for viewing, passing it through a circuit, and in a second successive and immediate washing cycle, a washing agent replacing the standard solution or liquid sample is passed through, thus leaving everything ready for carrying out a new calibration or analysis cycle, followed by a successive washing cycle.

2. A device for carrying out the method of claim 1, **characterized in that** it includes a test circuit with intake means for the liquid sample to be analyzed, some means (2, 3, 4, 5, 6, and 7) for containing the washing agent, alkalinizing solution, organic buffer compound solution and standard compositions; some means (8) for pumping the washing agent, alkalinizing solution, liquid sample and organic buffer compound solution; some first (9) and second (10) means of selection; a mixture chamber (11); a semipermeable membrane (12); an electrode (13) sensitive to the ammonium ion; a reference electrode (14); some electronic means for the treatment of the electrical signal; and representation means of the treated signal, all of this placed in such a way that, in a first calibration or analysis cycle, the first selection means take a small amount of alkalinizing solution and a small amount of standard composition or liquid sample respectively from the aforementioned containers means, which are pumped by the pumping means to the mixture chamber where they are mixed, and afterwards they are passed through the semipermeable membrane, the sensitive electrode and reference electrode, detecting a difference in potential which gives an electrical signal which is suitably treated by the electronic means and sent to the representation means where the concentration of total ammonium resulting from the analysis is digitally represented, then beeing carried out a washing cycle in which a washing agent is passed through the circuit in place of the standard composition or the liquid sample, leaving the device ready to carry out another calibration or analysis cycle, followed by a subsequent washing cycle.

3. A device according to claim 2, **characterized in that** the aforementioned first and second means of selection are each made up of two-way or more way valves (9, 10).

4. A device according to claim 2, **characterized in that** the aforementioned means of pumping are preferably made up of multi-channel peristaltic pump (8).

5. A method according to claim 1, **characterised in that** the aforementioned alkalinizing solution is an alkaline metals or alkaliearth hydroxide, with a concentration in the solution greater than 0.1M.

6. A method according to claim 1, **characterized in that** the aforementioned organic buffer compound is made up of weak monofunctional bases with a $pK_{acidity}$ of between 5 and 8.2 and with a concentration in the solution of between 0.01M and 0.15M.

7. A method according to claim 1, **characterized in that** the aforementioned standard calibration solutions are made up of any salt or combination of salts which contain the ammonium ion, particularly ammonium chloride.

8. A method according to claims 1 and 5 to 7, applicable to the measurement of urea in watery solutions in the event that the sample that must be analyzed contains ammonia in the form of urea, **characterized in that** this sample is first passed continuously through a reactor (37) which contains the urease enzyme, covalently immobilised in a suitable support, in a carrier solution which maintains a pH of between 7 and 8, so that the urea contained in the sample is converted into ammonium.

9. A method for measuring urea, according to claim 8, **characterized in that** the aforementioned carrier solution is a pH regulator solution adapted to maintain the pH in the order of 7.4.

10. A method according to claim 9, **characterized in that** the aforementioned pH regulator solution is made up of potassium phosphate-potassium hydrogenphosphate.

11. A method according to claim 9, **characterized in that** the aforementioned pH regulator solution is made up of sodium carbonate-bicarbonate.

12. A method according to claims 8 and 11, **characterized in that** a relatively high flow of sodium hydroxide solution is incorporated into the urea to ammonium degradation reaction system.

**Patentansprüche**

1. Verfahren zum Messen der Konzentration an gesamtem Ammonium in einem flüssigen Medium, **dadurch gekennzeichnet, dass** in einem ersten Kalibrierungs- oder Analysenzyklus eine Mischung aus einer Alkalisierungslösung und einer Standard-Kalibrierungslösung bzw. eine zu analysierende Flüssigprobe durch die Stirnseite (12a) einer halbdurchlässigen Membran (12) geschickt wird, während durch die andere Stirnseite (12b) der vorgenannten halbdurchlässigen Membran eine Lösung einer organischen Pufferverbindung fliessen gelassen wird, so dass die Mischung Ammoniak in einer Gasphase abgibt, welche beim Fliessen durch die halbdurchlässige Membran zu Ammonium rückumgewandelt wird, das durch eine Elektrode (13), die gegenüber dem Ammoniumion empfindlich ist, geleitet wird, welche ein Potential bildet, das zu dem Logarithmus der Konzentration an vorhandenen Ammoniumionen proportional ist, und dieses Potential wird mit dem von einer Referenzelektrode (14) erhaltenen verglichen, worauf der zwischen beiden Elektroden gemessene Potentialunterschied verstärkt wird und in ein elektrisches Signal umgewandelt wird, welches zum Betrachten in geeigneter Weise modifiziert wird, durch Hindurchschicken durch einen Stromkreis, und in einem zweiten sukzessiven und unmittelbaren Waschzyklus wird ein Waschmittel, welches die Standardlösung oder Flüssigprobe ersetzt, hindurchgeleitet, wodurch alles zur Durchführung eines neuen Kalibrierungs- oder Analysenzyklus in Bereitschaft bleibt, gefolgt von einem sukzessiven Waschzyklus.

2. Vorrichtung zur Durchführung des Verfahrens gemäss Anspruch 1, **dadurch gekennzeichnet, dass** es einen Testkreislauf mit Einlasseinrichtungen für die zu analysierende Flüssigprobe, einige Einrichtungen (2, 3, 4, 5, 6 und 7) für die Aufnahme des Waschmittels, der Alkalisierungslösung, der organischen Pufferverbindungslösung und der Standardzusammensetzungen; einige Einrichtungen (8) zum Pumpen des Waschmittels, der Alkalisierungslösung, der Flüssigprobe und der organischen Pufferverbindungslösung; einige erste (9) und zweite (10) Einrichtungen der Wahl; eine Mischkammer (11); eine halbdurchlässige Membran (12); eine Elektrode (13), die gegenüber dem Ammoniumion empfindlich ist; eine Referenzelektrode (14); einige elektronische Einrichtungen zur Behandlung des elektrischen Signals; und Darstellungseinrichtungen des behandelten Signals einschliesst, alles angeordnet in einer Weise, dass in einem ersten Kalibrierungs- oder Analysenzyklus die ersten Auswahleinrichtungen eine kleine Menge der Alkalisierungslösung und eine kleine Menge der Standardzusammensetzung bzw. der Flüssigprobe aus den vorgenannten Behältereinrichtungen, die durch die Pumpeinrichtungen zu der Mischkammer gepumpt werden, wo sie gemischt werden, aufnehmen und anschliessend diese durch die halbdurchlässige Membran, die empfindliche Elektrode und die Referenzelektrode geleitet werden, wobei ein Potentialunterschied festgestellt wird, welcher zu einem elektrischen Signal führt, welches in geeigneter Weise durch die elektronischen Einrichtungen behandelt wird und an die Darstellungseinrichtungen geschickt wird, wo die Konzentration des gesamten Ammoniums, die aus der Analyse resultiert, digital wiedergegeben wird, im Anschluss ein Waschzyklus durchgeführt wird, in welchem ein Waschmittel durch den Kreislauf an Stelle der Standardzusammensetzung oder der Flüssigprobe geleitet wird, wobei die Vorrichtung in Bereitschaft bleibt zur Durchführung eines weiteren Kalibrierungs- oder Analysenzyklus, gefolgt von einem nachfolgenden Waschzyklus.

3. Vorrichtung gemäss Anspruch 2, **dadurch gekennzeichnet, dass** die vorgenannten ersten und zweiten Auswahleinrichtungen jeweils aus Zweiweg- oder Mehrwegventilen (9, 10) bestehen.

4. Vorrichtung gemäss Anspruch 2, **dadurch gekennzeichnet, dass** die vorgenannten Pumpeinrichtungen vorzugsweise aus einer Mehrkanal-Peristaltikpumpe (8) bestehen.

5. Verfahren gemäss Anspruch 1, **dadurch gekennzeichnet, dass** die vorgenannte Alkalisierungslösung eine Alkalimetall- oder Erdalkalihydroxid ist mit einer Konzentration in der Lösung von höher als 0,1M.

6. Verfahren gemäss Anspruch 1, **dadurch gekennzeichnet, dass** die vorgenannte organische Pufferverbindung aus schwachen monofunktionellen Basen mit einer $pK_{Acidität}$ zwischen 5 und 8,2 und mit einer Konzentration in der Lösung zwischen 0,01M und 0,15M besteht.

7. Verfahren gemäss Anspruch 1, **dadurch gekennzeichnet, dass** die vorgenannten Standard-Kalibrierungslösungen aus irgendeinem Salz oder einer Kombination aus Salzen bestehen, die das Ammoniumion, insbesondere Ammoniumchlorid, enthalten.

8. Verfahren gemäss den Ansprüchen 1 und 5 bis 7, anwendbar auf die Messung von Harnstoff in wässrigen Lösungen für den Fall, dass die Probe, die analysiert werden muss, Ammoniak in der Form von Harnstoff enthält, **dadurch gekennzeichnet, dass** diese Probe zuerst kontinuierlich durch einen Reaktor (37) geleitet wird, welcher das Ureaseenzym enthält, kovalent immobilisiert in einem geeigneten Träger, in einer Trägerlösung, welche einen pH-Wert zwischen 7 und 8 aufrechterhält, so dass der in der Probe enthaltene Harnstoff zu Ammonium umgewandelt wird.

9. Verfahren zum Messen von Harnstoff gemäss Anspruch 8, **dadurch gekennzeichnet, dass** die vorgenannte Trägerlösung eine pH-Regulierlösung ist, die so ausgelegt ist, dass der pH-Wert in einem Grössenbereich von 7,4 gehalten wird.

10. Verfahren gemäss Anspruch 9, **dadurch gekennzeichnet, dass** die vorgenannte pH-Regulierlösung aus Kaliumphosphat-Kaliumhydrogenphosphat besteht.

11. Verfahren gemäss Anspruch 9, **dadurch gekennzeichnet, dass** die vorgenannte pH-Regulierlösung aus Natriumcarbonat/-bicarbonat besteht.

12. Verfahren gemäss Anspruch 8 und 11, **dadurch gekennzeichnet, dass** eine relativ hohe Strömung an Natriumhydroxidlösung in das Harnstoff-zu-Ammonium-Zersetzungs-Reaktionssystem eingebracht wird.

## Revendications

1. Procédé de mesure de la concentration totale d'ammonium dans un milieu liquide, **caractérisé en ce que**, dans un premier cycle d'étalonnage ou d'analyse, on fait passer respectivement un mélange d'une solution d'alcalinisation et d'une solution étalon pour l'étalonnage ou un échantillon de liquide à analyser à travers la face (12a) d'une membrane semi-perméable (12), tandis que l'on fait passer à travers l'autre face (12b) de la membrane semi-perméable susmentionnée, une solution d'un composé tampon organique de sorte que le mélange dégage de l'ammoniac en phase gazeuse, qui lors de son passage à travers la membrane semi-perméable est reconverti en ammonium, que l'on fait passer à travers une électrode (13) sensible à l'ion ammonium, laquelle engendre un potentiel proportionnel au logarithme de la concentration en ions ammonium présents, et on compare ce potentiel à celui obtenu à partir d'une électrode de référence (14), à la suite de quoi la différence de potentiel mesurée entre les deux électrodes est amplifiée et convertie en un signal électrique que l'on modifie de manière appropriée en vue d'une visualisation, en le faisant passer à travers un circuit, et dans un deuxième cycle de lavage successif et immédiat, on fait passer un agent de lavage remplaçant la solution étalon ou l'échantillon liquide en laissant ainsi le tout prêt à effectuer un nouveau cycle d'étalonnage ou d'analyse, suivi par un cycle de lavage successif.

2. Dispositif pour réaliser le procédé selon la revendication 1, **caractérisé en ce qu'**il comprend un circuit d'essai avec un moyen d'admission pour l'échantillon liquide à analyser, des moyens (2,3,4,5,6 et 7) pour contenir l'agent de lavage, la solution d'alcalinisation, la solution de composé tampon organique et les compositions étalons; certains moyens (8) pour pomper l'agent de lavage, la solution d'alcalinisation, l'échantillon liquide et la solution de composé tampon organique; des premier (9) et deuxième (10) moyens de sélection; une chambre de mélange (11); une membrane semi-perméable (12); une électrode (13) sensible à l'ion ammonium; une électrode de référence (14); des moyens électroniques pour traiter le signal électrique; et des moyens pour représenter le signal traité, le tout placé d'une telle façon que dans un premier cycle d'étalonnage ou d'analyse, le premier moyen de sélection prélève respectivement une petite quantité de solution d'alcalinisation et une petite quantité de composition étalon ou d'échantillon liquide à partir des moyens formant récipients susmentionnés, lesquelles sont pompées par le moyen de pompage vers la chambre de mélange où elles sont mélangées, puis on les fait passer à travers la membrane semi-perméable, l'électrode sensible et l'électrode de référence, en détectant une différence de potentiel qui donne un signal électrique qui est traité de manière appropriée par les moyens électroniques et qui est transmis vers les moyens de représentation où la concentration totale en ammonium résultant de l'analyse est représentée de façon numérique, puis en réalisant un cycle de lavage dans lequel on fait passer un agent de lavage à travers le circuit à la place de la composition étalon ou de l'échantillon liquide, laissant le dispositif prêt à effectuer un autre cycle d'étalonnage ou d'analyse, suivi par un cycle de lavage ultérieur.

3. Dispositif selon la revendication 2, **caractérisé en ce que**, les premier et deuxième moyens de sélection susmentionnés sont chacun constitués de vannes (9,10) à deux ou plusieurs voies.

4. Dispositif selon la revendication 2, **caractérisé en ce que** les moyens de pompage susmentionnés sont constitués de préférence d'une pompe péristaltique multivoie (8).

5. Procédé selon la revendication 1, **caractérisé en ce que** la solution d'alcalinisation susmentionnée est celle d'un hydroxyde de métaux alcalins ou alcalinoterreux avec une concentration supérieure à 0,1 M dans la solution.

6. Procédé selon la revendication 1, **caractérisé en ce que**, le composé tampon organique susmentionné est constitué de bases faibles monofonctionnelles ayant un $pK_{acidité}$ compris entre 5 et 8,2 et ayant une concentration comprise entre 0,01 M et 0,15 M dans la solution.

7. Procédé selon la revendication 1, **caractérisé en ce que**, les solutions étalons susmentionnées pour l'étalonnage sont constituées de tout sel ou combinaison de sels qui contiennent l'ion ammonium, en particulier le chlorure d'ammonium.

8. Procédé selon l'une quelconque des revendications 1 et 5 à 7, applicable à la mesure de l'urée dans des solutions aqueuses dans le cas où l'échantillon que l'on doit analyser, contient de l'ammoniac sous la forme d'urée, **caractérisé en ce que** l'on fait d'abord passer en continu cet échantillon à travers un réacteur (37) qui contient l'enzyme uréase, immobilisée par une liaison covalente sur un support approprié, dans une solution véhicule qui maintient un pH compris entre 7 et 8, de sorte que l'urée contenue dans l'échantillon est convertie en ammonium

9. Procédé de mesure de l'urée, selon la revendication 8, **caractérisé en ce que**, la solution véhicule susmentionnée est une solution régulatrice du pH adaptée pour maintenir le pH à une valeur de l'ordre de 7,4.

10. Procédé selon la revendication 9, **caractérisé en ce que** la solution régulatrice du pH susmentionnée est constituée de phosphate de potassium/hydrogénophosphate de potassium.

11. Procédé selon la revendication 9, **caractérisé en ce que** la solution régulatrice du pH susmentionnée est constituée de bicarbonate/carbonate de sodium.

12. Procédé selon la revendication 8 et 11, **caractérisé en ce que** l'on incorpore un débit relativement élevé de solution d'hydroxyde de sodium dans le système réactionnel de dégradation de l'urée en ammonium.

FIG. 1

FIG. 2

FIG.3

FIG.4

FIG.5

$V_1$ (mV)

$C_{urea}$ ($10^n$M)

FIG. 6

$V_2$ (mV)

$C_{urea}$ ($10^n$M)

FIG. 7

FIG. 8